(19) Europäisches Patentamt

European Patent Office

Office européen des brevets

(11) **EP 2 879 075 A1**

(12) # EUROPEAN PATENT APPLICATION
published in accordance with Art. 153(4) EPC

(43) Date of publication:
**03.06.2015 Bulletin 2015/23**

(51) Int Cl.:
**G06K 7/00** (2006.01)

(21) Application number: **13822326.8**

(22) Date of filing: **12.07.2013**

(86) International application number:
**PCT/ES2013/070501**

(87) International publication number:
**WO 2014/016458 (30.01.2014 Gazette 2014/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **26.07.2012 ES 201231201**

(71) Applicant: **Consejo Superior De Investigaciones Científicas (CSIC)
28006 Madrid (ES)**

(72) Inventors:
• **DELGADO RESTITUTO, Manuel
E-41092 Sevilla (ES)**

• **RODRÍGUEZ RODRÍGUEZ, José Antonio
E-41092 Sevilla (ES)**
• **RUIZ AMAYA, Jesús
E-41092 Sevilla (ES)**
• **MASUCH, Jens
E-41092 Sevilla (ES)**
• **RODRÍGUEZ PÉREZ, Alberto
E-41092 Sevilla (ES)**

(74) Representative: **Pons Ariño, Angel et al
Pons Patentes y Marcas Internacional, S.L.
Glorieta Rubén Dario 4
28010 Madrid (ES)**

(54) **WIRELESS RANGE-FINDING SYSTEM FOR THE MONITORING OF STATIC AND DYNAMIC VARIABLES**

(57) The invention relates to a wireless range-finding system based on the transmission of a time-based references for the monitoring of static and dynamic variables. The system comprises a read instrument and at least one transponder, in turn including transducers associated with the variables being observed and/or measured. In order to communicate the read instrument and the transponder, the system also comprises a mechanism for transmitting information relating to the internal operating frequency of the transponder to the read instrument, and a method for remotely determining the internal operating frequency of the transponder. The red instrument comprises: an antenna (11000), a communication section (12000), a signal processing section (13000), a computation section (14000), a temperature-stable timing unit (15000), a user interface section (16000) and a data output section (17000).

10000

20000

INTERROGATOR

WIRELESS SENSOR

**FIG. 1**

EP 2 879 075 A1

**Description**

**Object of the invention.**

The present invention proposes a wireless telemetry system based on transmission of time reference for the monitoring of static and dynamic magnitudes. The proposed system comprises several aspects such as a reading instrument and, at least, a transponder which, in turn, includes the transducers associated to the variables object of observation and/or measurement. With the object of communicating the reading instrument and the transponder with each other, the present invention also comprises a mechanism for the transmission of information regarding the internal operating frequency of the transponder towards the reading instrument and a method for the remote determination of the internal operating frequency of the transponder.

The invention is included within the sector of physical technologies and, more specifically, in the field of information technologies and technologies applied to telemetrics.

**Background of the invention**

The creation of smart spaces demands the use of wireless systems, preferably with a low form factor, capable of remotely monitoring physical or chemical magnitudes, or of any other type, which can be observed in the environment. Such wireless systems have a reading instrument (also called interrogator), which serves to activate the measurement process and to process the information received; and a transponder with sensing capacity (also called wireless sensor), which is responsible for the wireless capturing and transmitting of the measurements of the variables under observation. Whilst the transponder with sensing capacity must be arranged in such a manner so as to guarantee the reliability of the measurements, the reading instrument can be kept at a certain distance, limited by the communication range between both apparatuses.

With the aim of extending the time of useful life of the transponder, favouring reuse thereof and reducing maintenance costs, it is desirable that the wireless sensor as a whole operates without batteries. It is also recommendable that the interrogator-transponder system has a wide wireless coverage so that a single reader, configured for said purpose, may monitor the measurements of different transponders, avoiding collisions between the different sources of information. Furthermore, it is convenient that, whenever the variables under observation so allow, the transponders may withstand the measurement of different magnitudes to, on the one hand, reduce the number of devices (lower implementation cost) and, on the other hand, decrease the wireless information traffic (greater energy response).

These requirements require the use of design techniques with ultra-low power consumption techniques, as well as, energy generators linked to the transponder, capable of obtaining supply voltages from environmental resources. They also demand the use of architectures and processes which allow the reuse of component blocks, if the transponder has to support the measurement of variables of a different nature. Also, and with a view to reducing the production cost, measurement and adjusting phases must be avoided during the manufacturing processes, i.e. means must be provided for self-calibration of the telemetry system. All of this must also be achieved satisfying the values of precision and resolution of the measurements typical of the application environment.

An example wherein said features are especially relevant is in the monitoring of physiological patterns in individuals. Clearly, the use of wireless technologies with transponders with sensing capacity which have reduced dimensions and are easily portable, would allow the remote reading of vital signs of the patient, without reducing the mobility or comfort levels thereof. For the same reason, the concentration in a single transponder of different sensors, such as, for example, body temperature, ECG patterns, muscular activity or breathing rate, would favour a simple and discrete monitoring of the patient.

This example from the healthcare field reveals one aspect common to most telemetry systems designed for the creation of smart spaces, i.e. the low complexity of the information to transmit as well as the reduced cadence of variation of the data (in the order of seconds). For this reason, and bearing in mind, furthermore, the demands of low consumption and the trend towards battery-free solutions, the techniques based on passive RFID technologies (Identification by Radiofrequency) constitute a promising alternative for the implementation of wireless telemetry systems. However, in most of the embodiments proposed to date, said systems only support the monitoring of a single variable under observation, instead of a multiple monitoring as would be desirable. Furthermore, said variables under observation are essentially static, so that monitoring dynamic properties is not contemplated. Even more so, in many cases, the sensors connected to the transponders must be supplied with batteries, so that it violates the objective of the passivity of the device which, preferably, must provide energy not only to the blocks linked to the wireless link, but also to the sensors wherefrom the magnitudes monitored are captured.

As in other fields of application, these limitations are evident in the telemetry systems for the wireless monitoring of existing physiological patterns which, do not satisfy all the above objectives at all. For example, the document of the state of the art with publication number WO-2011010437(A1) and title *"System for measuring patients body temperature*

*used in hospital, has data reading device which changes electric power level in excitation unit when magnetic field change is detected"* discloses a device to measure body temperature using sensors included in a processing unit but it does not contemplate the monitoring of multiple variables. The document of the state of the art with title "Full Passive UHF Tag with a Temperature Sensor Suitable for Human Body Temperature Monitoring" by A. Vaz, et al. (IEEE Trans. on Circuits and Systems II - Express Briefs, Vol. 57(2), Pages 95-99 DOI: 10.1109/TCSII.2010.2040314) also exclusively centres on the measurement of body temperature. Furthermore, as with WO-2011010437(A1), the system uses integrated circuits which require the use of calibration. The proposal disclosed in the patent application with publication number US-A1-2010156598 and title *"Radio frequency identification (RFID) medical device for reading physiological parameter e.g. temperature from patient, has transmitter for transmitting sensed signal from sensor to reading in contact less manner"* proposes the use of multiple sensor elements external to the RFID information transmission unit. However, said sensors must be individually supplied by batteries, so that the implementation is not completely passive. The contribution contained in the patent application with publication number US-A1-2010171596 and title *"In vivo complementary metal oxide semiconductor compatible radio frequency identification chip implanted in body of patient, has compatible circuitry that controls drug release from drug reservoir based on biosensed information"* uses passive RFID transponders, however, it is designed for the injection of drugs from implantable devices. Finally, the contribution of the patent application with publication number US-A1-2004215098 and title *"Skin patch including a temperature sensor"* is conceptually similar to US-A1-2010156598, and requires numerous discrete components (including two button batteries) which considerably increase the transponder form factor.

## Description of the invention

The present invention resolves the aforementioned problem and satisfies all described requirements. In accordance with the present invention, a wireless telemetry system is provided for the measurement of variables under observation and a method to perform said monitoring. Variable under observation is understood to be any property of a physical or chemical magnitude or of any other type which can be detected and converted by a transducer into an electrical magnitude which can be interpreted by the telemetry system. In accordance with the present invention, said electrical magnitudes which can be interpreted can be electrical signals or properties of circuit elements. Also in accordance with the present invention, the electrical magnitudes derived from the transducers may essentially be static or dynamic. In this last case, the variable under observation reflects any characteristic of the magnitude monitored such as, for example, frequency, rate of events, average value or, in general, any dynamic property computable within the measurement period.

In accordance with the present invention, the telemetry system comprises a reading instrument and, at least, a transponder which, in turn, includes the transducers associated to the variables under observation. In accordance with the present invention, every transponder stores an exclusive identification thereof which distinguishes it from the other transponders.

Likewise, in accordance with the present invention, the operation of the wireless telemetry system comprises three fundamental phases. In first place, the reading instrument selects and interrogates a transponder from among a limited array of transponders in accordance with the present invention, located in the coverage range of the communications. In second place, the selected transponder transmits the data required by the reading instrument in response to the interrogation signal received. In third place, the reading instrument interprets the information emitted by the transponder, and transfers the corresponding data to the user.

In accordance with the present invention, the interrogation signal emitted by a reading instrument may require information on one or several aspects. One possible aspect is the identification of the transponder. Another possible aspect is the internal operating frequency of the transponder. Even another possible aspect is the representative values of the measurements of one or more variables under observation performed by the transponder.

Also, in accordance with the present invention and irrespective of whether the electrical magnitude associated to the variable under observation is essentially static or dynamic, the representative value of the measurement is a binary coded number which unequivocally quantifies the magnitude monitored during the measurement time. Similarly, the representative value of the internal operating frequency of a transponder in accordance with the present invention is a digital word.

In accordance with the present invention, the binary numbers representative of the internal operating frequency of a transponder and of the measurements of the variables under observation are encoded as counts of cycles of a reference clock which autonomously operates in a transponder in accordance with the present invention.

In one aspect of the present invention, a reading instrument is provided which comprises: an antenna capable of transmitting different interrogation signals towards a transponder in accordance with the present invention and of receiving information signals from said transponder; a communication section connected to the antenna which provides the means and processes to define the signal formats, the data encoding, the modulation type, the transmission/reception rates, the RF envelope and, in general, all those parameters necessary to establish the wireless communication with a transponder in accordance with the present invention; a signal processing section connected to the communication section which provides the means and processes necessary to create, in accordance with the user's instructions, the command sequence required to select and interrogate a transponder in accordance with the present invention, as well as to identify and classify the data received from said transponder; a computation section which interprets the internal operating frequency data of the transponder and of measurement of the variable under observation, previously classified in the data processing section; a timing unit stable with temperature which determines the operating frequency of the entire reading instrument and which provides the computation section with the parameters necessary to interpret the representative values both of the internal operating frequency of the transponder and of the measurements of the variables under observation; a user interface section which provides the means to activate the identification and measurement processes of variables under observation from a transponder in accordance with the present invention, and a data output section which provides the means and processes to store the data measured from the transponders of the environment and of communicating said data with the exterior.

In another aspect of the present invention, it provides a transponder for a wireless monitoring system designed for the measurement of one or more variables under observation comprising: an antenna capable of receiving different interrogation signals from a reading instrument in accordance with the present invention and of transmitting to said reader signals representative of their exclusive identification and of its internal operating frequency, as well as of the measurements of the variables under observation; an active circuit which manages the communication operations with the reading instrument as well as the data processing operations in accordance with the instructions received; and an array of transducers, associated to the variables under observation, which convert the monitored magnitudes to electrical values (signal or circuit property) which can be interpreted by the active circuit. In accordance with the present invention, the transformation performed by each transducer is a monotone function, known and available in the computation section of a reading instrument in accordance with the present invention.

The active circuit comprised in the transponder of the present invention comprises: a storage section wherein a unique registration code is registered for said transponder; an energy generation and storage unit wherein the supply voltages necessary for operation of the transponder are generated and regulated; a timing section wherein the clock signals necessary to active the transponder are autonomously generated; a signal modulation and demodulation section wherein the binary codes included in the interrogation signals are extracted from a reading instrument in accordance with the present invention, and wherein the data generated by the transponder are modulated in accordance with the requests made by said reading instrument; a processing unit wherein the commands received from a reading instrument are interpreted in accordance with the present invention, the operating modes of the different sections of the active circuit are managed to undertake the actions included in said commands and format is given to the binary numbers representative of the internal operating frequency of a transponder and of the measurements of the variables under observation which must be sent to said reading instrument; and an acquisition and measurement section which provides the means and processes to sequence the activity of the transducers, convert the analogue signals obtained from said transducers to the digital domain and transfer said digital data to the processing unit. In accordance with the present invention, the data conversion performed in the acquisition and measurement section is a monotone function, known and available in the computation section of a reading instrument in accordance with the present invention.

The active circuit comprised in a transponder, in accordance with the present invention, is manufactured on a substrate chosen from among: silicon, organic, silicon on insulator, silicon-germanium, indium phosphide and gallium arsenide. Preferably, with a view to reducing the manufacturing cost of the device, silicon substrate is used. Likewise, if any of the transducers associated to the variables under observation may be integrated in silicon, said integration shall preferably be performed on the same substrate of the active circuit, with a view to favouring the miniaturization and the cost of the transponder.

Since, in accordance with the present invention, the transformations and conversions used to obtain the numbers representative of the variables under observation are unequivocal, invertible and known with sufficient accuracy; and since the encoding of such numbers is performed using time quantification using as reference the clock signal generated in the timing section of the active circuit in accordance with the present invention; the reading instrument only requires a precise estimate of the frequency to faithfully interpret the measurements of the variables under observation.

To perform the transmission of information regarding the internal operating frequency of the transponder towards the reading instrument, the present invention comprises a mechanism whereby the transponder comprises a finite state machine installed in the transponder which detects a timing frame sent from the reading instrument, and a digital counter activated by an internal clock of the transponder which counts the number of clock periods comprised in said frame. In a preferred embodiment of the mechanism to determine internal operating frequency of the transponder, both the state machine and the counter are included in the processing section of the active circuit comprised in the transponder of the present invention.

In another aspect of the invention a communication method is provided between the transponder and the reading instrument, both for a wireless telemetry system for the monitoring of static and dynamic magnitudes, where the transponder is defined in any one of the embodiments described for the transponder, and where the reading instrument is defined according to any one of the embodiments described for the reading instrument. The communication method comprises: in first place, the reading instrument selects and interrogates a transponder from among a limited array of transponders located in a coverage range of the reading instrument by the sending of an interrogation signal; in second place, the selected transponder transmits data required by the reading instrument in response to the interrogation signal received; in third place, the reading instrument receives and interprets the data emitted by the transponder and transfers data corresponding to the user. This process of request, response and interpretation of information can also be applied to any transponder within the range of the interrogator.

As part of the communication method between the transponder and the reading instrument, the present invention provides a form for remotely determining the internal operating frequency of the transponder by the reading instrument. The remote determination of the internal operating frequency of the transponder by the reading instrument is carried out using the following stages: generation in the reading instrument of a command containing a specific binary frame of predetermined duration; reception, demodulation and decoding in the transponder of the command emitted by the reading instrument; activation of a state machine to detect the initial and final instants of the specific binary frame; enabling of a digital counter, operated at a frequency proportional to the internal frequency of the transponder, at the moment when the state machine detects the initial instant of the frame; disabling of the counter when the state machine detects the final instant of the frame; storage, encoding and later sending to the reading instrument of the number of cycles counted by the digital counter; reception, demodulation and decoding of said number of cycles in the reading instrument; and calculation of the internal frequency of the transponder in accordance with the number of cycles counted, the proportionality constant of the clock used in the count and the duration of the binary frame sent by the reading instrument. In accordance with the present invention, the calculation of the internal operating frequency of the transponder is performed in the computation section of a reading instrument in accordance with the present invention.

In accordance with the present invention, the interpretation of the measurements of the variables under observation performed in the reading instrument is performed in accordance with the representative numbers sent and the estimate of the operating frequency of a transponder in accordance with a the present invention obtained from the previously described mechanisms.

Therefore, the wireless telemetry system for the monitoring of static and dynamic magnitudes comprises a transponder in accordance with any one of the aforementioned embodiments described for the transponder, a reading instrument according to any one of the aforementioned embodiments described for the reading instrument and a communication method between the transponder and the reading instrument also according to one of the embodiments described for the communication method. The system has, therefore, a unique inventive concept (telematically measure a static and/or dynamic magnitude) which provides unity of invention to the present application, as occurs in emitter-receiver systems.

It is important to highlight that the concepts and specifications described in the present invention are general and are not strictly linked to any type of wireless communications standard in particular.

In the context of the present invention, the terms "approximately" or "in the order of" must be understood as indicating values very close to those that said term accompanies. The person skilled in the art will understand that a small deviation in the values indicated, within reasonable terms, is inevitable due to measurement inaccuracies, etc.

**Brief description of the figures.**

With the object of complementing the description of the invention and its characteristics, the following figures are attached as an integral part of said description.

Figure 1 represents a general block diagram of the wireless monitoring system of patients' body temperature and heart rate, comprising an "INTERROGATOR" reading instrument and transponder "WIRELESS SENSOR".

Figure 2 represents a general block diagram of the "INTERROGATOR" of the wireless monitoring system of patients' body temperature and heart rate, comprising an "ANTENNA", a communication section "SIGNAL MODULATION AND FORMAT", a data processing section "SIGNAL PROCESSING", an arithmetic computation section "COMPUTE", a clock signal generation and management section "TIMING", a module for the presentation of results to the user "INTERFACE" and a unit for external storage and communication "DATA OUTPUT".

Figure 3 represents the block diagram of the "SIGNAL PROCESSING" section of the "INTERROGATOR" comprising a "CONFIGURATION LOG", a digital data processor "PROCESSOR", a command generator which encapsulates the parameters generated by the processor in frames in accordance with the communication standard "COMMAND GENERATOR" and a data input/output block "DATA I/O".

Figure 4 represents the block diagram of the "CALCULATION" section of the "INTERROGATOR" comprising an arithmetic-logic unit "ALU", an intermediate operations log "OPERATION LOG" and a final result storage log "RESULT LOG".

Figure 5 shows an example of signal format for symbols '0' and '1' obtained from the master clock CLKL available in the "INTERROGATOR". In this example, the parameters M0 and M1 take the values 8 and 16, respectively, in accordance with the number of clock cycles CLKL that occupy symbols '0' and '1',

Figure 6 represents the general block diagram of the transponder "WIRELESS SENSOR" of the wireless monitoring system of patients' body temperature and heart rate, comprising an "ANTENNA", a transponder integrated circuit "IC", an array of discrete components for the acquisition of body temperature and heart rate signals "DISCRETE INTERFACE COMPONENTS" and an array of discrete reference components "DISCRETE REFERENCE COMPONENTS".

Figure 7 represents the general block diagram of the transponder integrated circuit "IC" included in the transponder "WIRELESS SENSOR" comprising an energy management unit "ENERGY MANAGEMENT", a signal acquisition section "SIGNAL ACQUISITION", a timing unit "TIMING", a code and parameter storage section "STORAGE MEMORY", a data processing section "PROCESSING" and a signal encoding/decoding and modulation/demodulation section "SIGNAL DE/MODULATION DECODING/ENCODING".

Figure 8 shows a representation of the clock signals derived in the timing unit "TIMING", comprised in the transponder integrated circuit "IC".

Figure 9 represents the block diagram of the data processing unit "PROCESSING" included in the transponder integrated circuit "IC" comprising a frequency computation block "FREQUENCY COMPUTATION", an array of state machines responsible for processing the commands received and managing the corresponding responses "COMMAND FSMs"; an arithmetic logic unit "ALU"; and an array of logs for temporary information storage "LOGS".

Figure 10 represents the block diagram of the frequency computation unit "FREQUENCY COMPUTATION" included in the data processing section "PROCESSING" of the transponder integrated circuit "IC" comprising a counter "COUNTER", a state machine "FSM CONTROL" and a storage log "LOG".

Figure 11 shows the structure of an internal operating frequency request command of the transponder integrated circuit "IC" generated by the reading instrument "INTERROGATOR".

Figure 12 shows the structure of the binary response sequence of the transponder integrated circuit "IC" when faced with an internal operating frequency request made by the reading instrument "INTERROGATOR".

Figure 13 shows two possible scenarios of wireless communication link between the reading instrument "INTERROGATOR" and the transponder "WIRELESS SENSOR" to obtain a remote measurement of the internal operating frequency of the transponder.

Figure 14 shows the structure of a WRITE command, in accordance with standard ISO/IEC 18000-6, configured for the measurement start request in a transponder "WIRELESS SENSOR", in accordance with an example of embodiment of the present invention.

Figure 15 shows the structure of a binary response frame, in accordance with standard ISO/IEC 18000-6, to a WRITE command configured for the measurement start request in a transponder "WIRELESS SENSOR", in accordance with an example of embodiment of the present invention.

Figure 16 shows the structure of a READ command, in accordance with standard ISO/IEC 18000-6, configured for the measurement sending request from a transponder "WIRELESS SENSOR", in accordance with an example of embodiment of the present invention.

Figure 17 shows the structure of a binary response frame, in accordance with standard ISO/IEC 18000-6, to a READ command configured for the measurement sending request from a transponder "WIRELESS SENSOR", in accordance with an example of embodiment of the present invention.

Figure 18 shows the basic steps included in a communication method between a transponder "WIRELESS SENSOR" and a reading instrument "INTERROGATOR", in accordance with an example of embodiment of the present invention.

Figure 19 shows the steps included in a method for the remote estimate of the internal operating frequency of a transponder "WIRELESS SENSOR" from a reading instrument "INTERROGATOR", in accordance with an example of embodiment of the present invention.

## Description of an example of embodiment of the invention

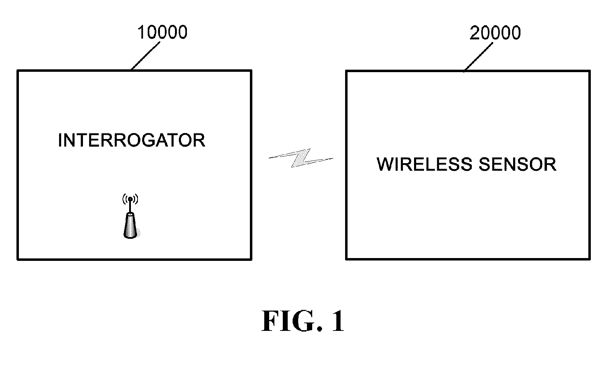

Figure 1 represents the basic blocks of the wireless telemetry system based on transmission of time reference for the monitoring of static and dynamic magnitudes, object of the present invention. The purpose of this system is the remote measurement of one or more variables under observation. In the context of the present invention, irrespective of the application environment, the term variable under observation (or variable monitored or monitored variable) makes reference to the property of a physical or chemical magnitude or of any other type, which may be detected by a transducer. Likewise, in the context of the present invention, a transducer is a device capable of converting the magnitude monitored into an electrical magnitude which can be interpreted by the telemetry system. In accordance with the present invention, the variables under observation may be, for example, of mechanical, thermal, optical, electromagnetic, chemical or biological type.

In accordance with the present invention, the electrical magnitudes which can be interpreted by the telemetry system may be electrical signals, such as flows of current or potential differences (for example, electrical voltage in Hall probes or electrical current in photodiodes); or properties of circuit elements, such as resistances, capacities or inductances (for example, electrical resistance in photoelectric cells or electrical capacity in accelerometers). Also in accordance with the present invention, the electrical magnitudes derived from the transducers may essentially be static or dynamic, depending on whether the transduction time constants are higher or lower than the total duration of the remote measurement process.

The telemetry system comprises a reading instrument 10000 (also called interrogator) and, at least, a transponder 20000 (also called wireless sensor) comprising in turn the transducers associated to the variables under observation. In accordance with the present invention, any transponder 20000 stores an exclusive identification thereof which distinguishes it from the other transponders. Likewise, in accordance with the present invention, the operation of the wireless telemetry system comprises three fundamental phases. In first place, the reading instrument 10000 selects and interrogates a transponder 20000 from among a limited array of transponders located in the coverage range of the communications. In second place, the selected transponder 20000 transmits the data required by the reading instrument 10000 in response to the interrogation signal received. In third place, the reading instrument 10000 interprets the information emitted by the transponder 20000, and transfers the corresponding data to the user. This request process, response and interpretation of information can also be applied to any transponder 20000 within the range of the interrogator 10000.

As regards the present invention, the interrogation signal emitted by a reading instrument 10000 may require information on one or several aspects. One possible aspect is the identification of the transponder 20000, potentially established throughout the manufacturing process thereof. Another possible aspect is the internal operating frequency of the transponder 20000. Even, another possible aspect is the representative values of the measurements of one or more variables under observation performed by the transponder 20000. In a possible implementation of the telemetry system object of the present invention, the flow of information between the reading instrument 10000 and the transponder 20000 may include other aspects in addition to those mentioned.

In accordance with the present invention, if the electrical magnitude associated to the variable under observation is essentially static, the representative value of the measurement is a number which unequivocally determines the monitored magnitude. For example, if the variable under observation is the mechanical stress whereto an object is subjected, the electrical magnitude interpretable by the telemetry system could be the resistance through a piezoresistant membrane disposed for said purpose, and the representative value of the measurement could be a number which informs of the deviation of the resistance of said membrane to the resistance insensitive to tractions or compressions. On the other hand, if the electrical magnitude associated to the variable under observation is dynamic, the representative value of the measurement is a number which unequivocally informs on a characteristic of said magnitude such as, for example, frequency, average value or, in general, any property computable within the measurement period. For example, if the variable under observation is the radioactivity level of an object or place, the electrical magnitude interpretable by the telemetry system could be the pulsed current generated by a particle detected, and the representative value of the measurement would consist of the number of pulses observed throughout the pre-established measurement time.

In accordance with processes which will be described later in detail, the binary numbers representative of the measurements of the variables under observation are encoded as counts of cycles of a reference clock which autonomously operates in a transponder 20000 in accordance with the present invention.

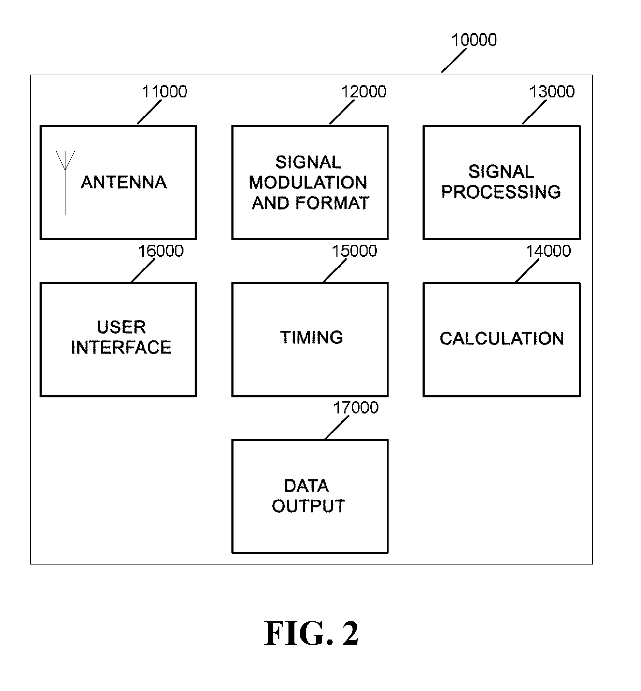

Figure 2 presents the block diagram of the reading instrument 10000 according to an embodiment of the present invention. The reading instrument of figure 2 comprises different elements and sections, 11000-17000.

The element 11000 is an antenna capable of transmitting different interrogation signals towards the transponder 20000 and of receiving information signals from the transponder 20000. In a preferred configuration, the antenna 11000 is of omnidirectional type.

Block 12000 represents a communication section connected to the antenna 11000 which provides the means and processes to define the signal formats, the data encoding, the modulation type, the transmission/reception rates, the RF envelope and, in general, all those parameters necessary to establish the wireless communication with a transponder 20000 in accordance with the present invention.

Block 13000 represents a signal processing section connected to the communication section 12000 which provides the means and processes necessary to: configure and control the state of the wireless link between the reading instrument 10000 and the transponder 20000 in accordance with the communication protocol adopted; create, in accordance with the user's instructions, the command sequence required to select and interrogate a transponder 20000 in accordance with the present invention; and identify and classify the data received from said transponder 20000. Furthermore, the signal processes section 13000 implements specific instructions for the request, reception and classification of information regarding the internal operating frequency of the transponder 20000, as well as, of information regarding the measurements of the variables monitored by said transponder.

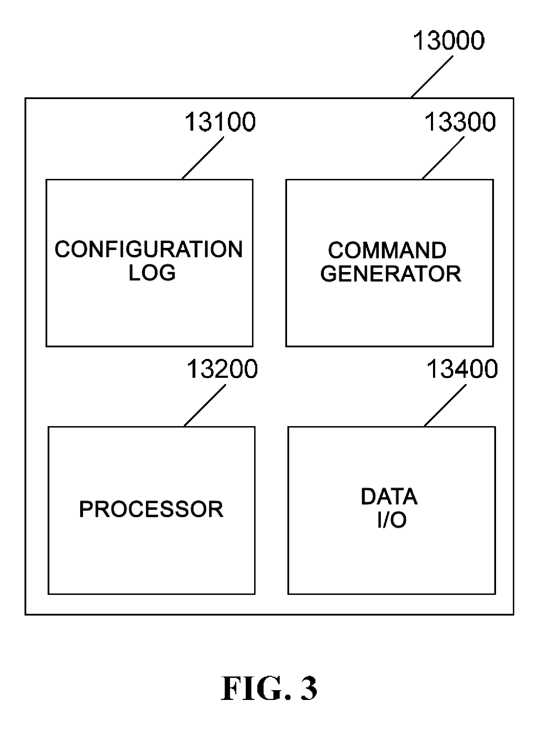

Figure 3 presents the block diagram of the processing section 13000 according to an embodiment of the present invention. Said section comprises a CONFIGURATION LOG 13100 wherein are stored the parameters of the wireless link between the reading instrument 10000 and the transponder 20000 selected; a processor 13200 which, in reception mode, interprets and responds to the binary flow decoded by block 12000 and, in transmission mode, provides the parameters which must be sent to the transponder 20000; a command generator 13300 which encapsulates the parameters generated by the processor 13200 in binary frames in accordance with the communication standard used; and a data input/output block 13400 wherein information is transferred towards/from other sections of the reading instrument 10000.

Block 14000 represents a computation section connected to the data processing section 13000 which provides the means and processes necessary both to interpret the representative values of the internal operating frequency of the transponder 20000, and to interpret the representative values of the measurements of each one of the variables monitored by said transponder. Preferably, the computation section 14000 remains inactive except when the processing section 13000 provides one or several of the data cited.

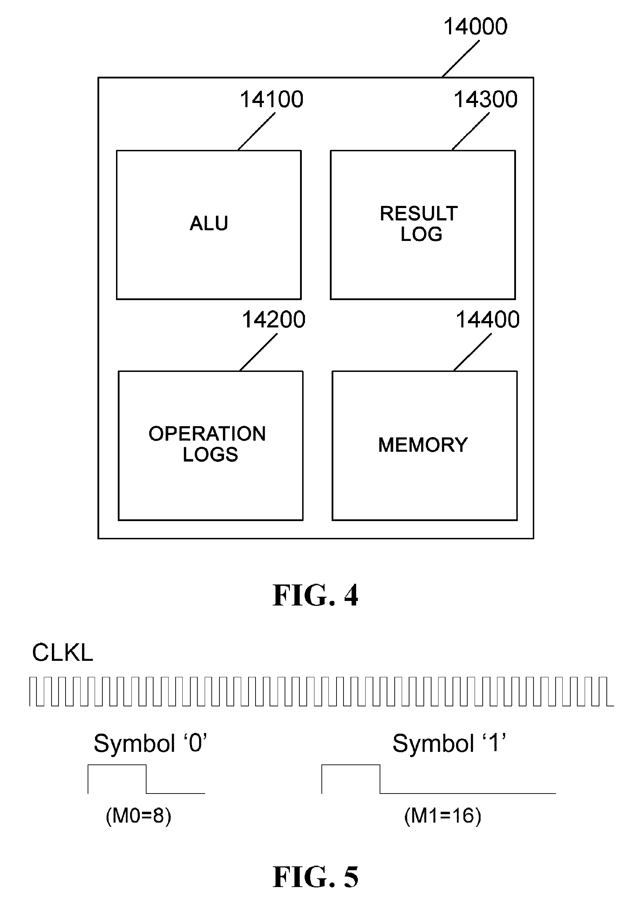

Figure 4 shows the block diagram of the computation section 14000 according to an embodiment of the present invention. Said section comprises an arithmetic logic unit (ALU) 14100 where the arithmetic operations necessary to calculate the internal operating frequency of the transponder 20000 are performed and to calculate the value of the measurements of the variables under observation; operation logs 14200 where the intermediate results of the operations, such as multiplications or sums are stored; a data log 14300 storing the final result generated by the ALU 14100; and an internal memory 14400 where all those numerical parameters are located necessary for the representative values classified as internal operating frequency of the transponder 20000 or as measurement of the variable under observation.

Block 15000 represents a timing unit stable with temperature which determines the operating frequencies of all the sections of the reading instrument and which provides the computation section 14000 with the time references necessary both to interpret the representative values of the internal operating frequency of the transponder 20000 and to interpret the information regarding the measurements of each one of the variables monitored by said transponder.

In a possible embodiment of the present invention, the operating frequencies of the reading instrument are very precisely derived from a master clock CLKL based on quartz crystal. The operating frequency of this master clock CLKL is FLEC. Optionally, this master clock CLKL may comprise a type of stabilization correction with temperature. Of special relevance in the context of the present invention, the frequency FLEC is used to define the duration, potentially different, of the binary symbols '0' and '1', in accordance with the signal format defined in section 12000 of the reading instrument 10000. In particular, the durations of the symbol '0', TLEC0, and of the symbol '1', TLEC1, are generically given by the expressions,

$$TLEC0 = M0 \: / \: FLEC \qquad (1)$$

$$TLEC1 = M1 \: / \: FLEC \qquad (2)$$

where M0 and M1 represent, respectively, the whole number of master clock cycles with frequency FLEC which occupy the symbols '0' and '1'. Figure 5 shows an example of representation of the symbols '0' and '1' derived from the master clock CLKL. In this example, with purely illustrative character, the parameters M0 and M1 take, respectively, the values 8 and 16.

Block 16000 represents a user interface section which provides the means so that an agent external to the wireless telemetry system (individual or machine) may activate the identification and measurement processes of the variables under observation from a transponder 20000 in accordance with the present invention. In a configuration of the present invention, the reading instrument 10000 is a portable apparatus which includes in the user interface section 16000 devices such as keyboards or sensors/touch screens, wherethrough the user orders can be transferred to the interrogator. Alternatively, or in conjunction with the above, the user interface section 16000 of the reading instrument 10000 has at least one external connection, wireless or by cable, wherethrough the identification and measurement processes available in the wireless telemetry system can be remotely activated in accordance with the present invention.

Finally, block 17000 represents a data output section which provides the means and processes to store the data measured from each transceiver 20000 of the environment and of communicating said data with the exterior. In a preferred configuration of the reading instrument 10000, the data output section 17000 comprises a screen to show the user the information received from the transponders. Alternatively, or in conjunction with the above, the output section of the reading instrument 17000 has at least an external connection, wireless or by cable, to send the information received by the transponders to an external storage and processing means.

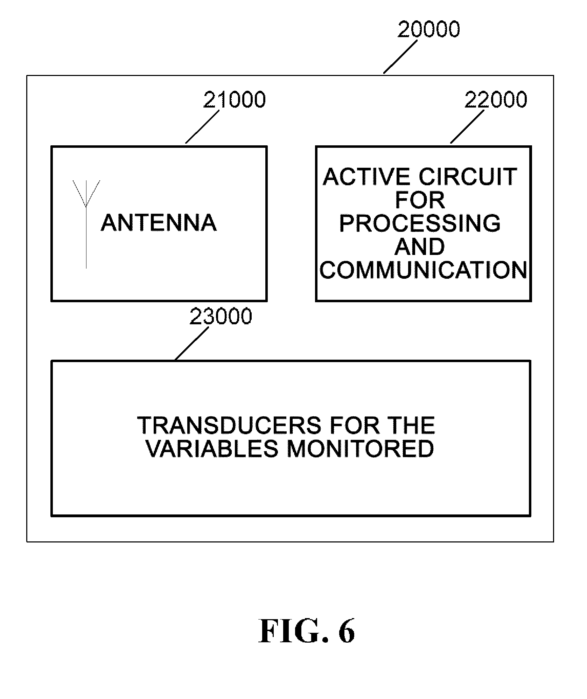

Figure 6 presents the block diagram of the transponder 20000 according to an embodiment of the present invention. The transponder of figure 6 comprises different elements and sections, 21000-23000.

The element 21000 is an antenna capable of receiving different interrogation signals from a reading instrument 10000 in accordance with the present invention and of transmitting to said reader 10000 the identification of the transponder 20000 and signals representative of the internal operating frequency of the transponder 20000, as well as of the measurements of the variables under observation.

Block 22000 is an active circuit which manages the communication operations with the reading instrument 10000 as well as the data processing operations in accordance with the instructions received. This block comprises, among other elements, the means necessary to store the identification of the transponder 20000 and the means and processes necessary to obtain the representative values of the internal operating frequency of the transponder 20000, as well as, of the measurements of the variables under observation.

In a preferred configuration of the present invention, the active block 22000 is integrated in a single microchip manufactured on a substrate chosen from among: silicon, silicon on insulator, silicon-germanium, indium phosphide and gallium arsenide. Preferably, with a view to reducing the manufacturing cost of the device, silicon substrate is used.

Section 23000 comprises the transducers associated to the variables under observation. In a broad sense, such transducers provide the means which implement the interface between the magnitudes wherefrom are derived the variables under observation and the active circuit for data and communications processing 22000. In accordance with this interpretation, the transducers contained in section 23000 comprise means of inspection or contact with the magnitude under observation, means of electrical interconnection with the active circuit 22000 and, possibly, active or passive means, integrated or based on discrete components, which transform the monitored magnitude into an electrical magnitude (electrical signal or circuit property) which can be captured and conditioned by the active block 22000. Said conversion must be monotone in the operating range defined by the application, so that there is a one-to-one correspondence between both magnitudes. In mathematical form, if $A_X$ represents the magnitude monitored by the transducer x, where x is a generic index which identifies each one of the transducers comprised in section 23000, and $E_X$ is the electrical magnitude derived from the transduction, both amounts are related by a bijective function $T_X(\bullet)$, so that,

$$E_X = T_X (A_X;\ C_X) \qquad\qquad (3)$$

In accordance with the present invention, the function Tx(•) is an intrinsic propriety of the transducer x, so that it is known and available in the computation section 14000 of the reading instrument 10000, either in the form of mathematical operations (executable in the arithmetic logic unit 14100), or in the form of tables (stored in the internal memory 14400). In the expression (3), Cx is an array of adjusting parameters required by the reading instrument 10000 for the correct interpretation of the representative value of the measurement of the variable under observation linked to the transducer x. Said parameters are stored in the transceiver 20000 (in particular in the memory module 22400 of the active block 22000) and they have to transmit to the reading instrument 10000 using means similar to those used for sending the identification of the transponder 20000.

In a preferred configuration of the present invention, if any of the transducers of section 23000 may be integrated in silicon, said integration is performed on the same substrate of the active block 22000, in the event this has been implemented using a single microchip. The objective is to create a SoC (System-on-Chip) which favours the miniaturization and the cost of the transponder 20000.

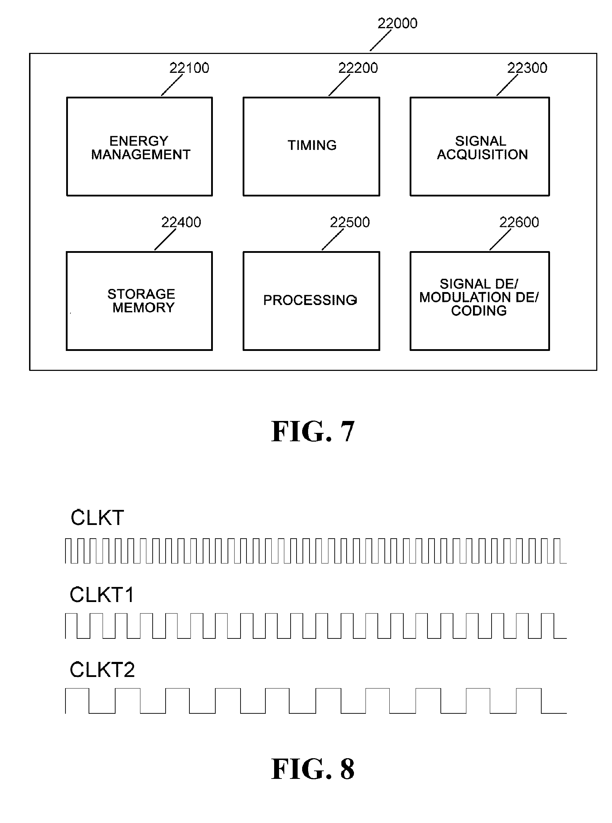

Figure 7 presents the block diagram of the active circuit 22000 according to an embodiment of the present invention. The active circuit of figure 7, preferably integrated in silicon, comprises different elements and sections, 22100-22600.

Block 22100 is an energy management unit wherein are generated and regulated the supply voltages necessary for the operation of the active circuit 22000. This unit generates at least positive and negative supply rails for the analogue circuit and for the digital circuitry. The energy management unit and, hence, the entire active circuit 22000, may be supplied by an external battery or, preferably, it may include the means and processes to generate and store energy from the signal radiated by the reading instrument 10000 and captured by the antenna 21000 of the transponder 20000. This last solution is in no way limitative of the present invention, but it can be complemented with other energy sources based on batteries or from environmental resources.

Block 22200 is a timing section wherein the clock reference signal CLKT, necessary to activate the data processing and coordination of the means in the active circuit 22000 is generated. The clock reference signal CLKT is a periodic pulse train at a frequency FTAG. In a preferred configuration of the present invention, the reference clock CLKT is obtained using an autonomous oscillator with compensation against temperature variations.

Together with the signal CLKT, the timing block 22200 also generates other clock signals of lower frequency, CLKTx, necessary for the digital encoding of the measurements of the variables under observation, in accordance with the present invention. As previously indicated, the generic index x takes different values in accordance with each one of the variables monitored by the transponder 20000. The clock signals CLKTx with frequency FTx are obtained by scaling the clock CLKT, so that:

$$FT_X = FTAG / MT_X \qquad (4)$$

where MTx are predefined whole constants associated to each transducer. In a possible embodiment, the scaling of the clock CLKT, to obtain the signals CLKTx, is implemented using digital synchronous counters. Figure 8 shows an example wherein two signals CLKT1 and CLKT2, necessary for the digital encoding of the measurements of the variables under observation 1 and 2, are derived using the division in frequency of the reference clock CLKT by factors of scale MT1=2 and MT2=4, respectively.

Furthermore, with a view to the transmission of a signal representative of the internal operating frequency of the transponder 20000, the timing section 22200 also provides a clock signal CLKCO whose period is a whole multiple MCO of the period of the reference signal CLKT, so that

$$FCO = FTAG / MCO \qquad (5)$$

where FCO is the frequency of the signal CLKCO.

Block 22300 is an acquisition and measurement section comprising, in accordance with the present invention, means and processes for the capture and conditioning of the electrical magnitudes provided by the transducers of section 23000 and, means and processes for the derivation and digitization of the representative values of the measurements of the variables under observation.

On the one hand, in accordance with the present invention, the means and processes of the acquisition and measurement block 22300 designed for the capture and conditioning of the electrical magnitudes provided by the transducers of section 23000 have the objective of the synthesis of an equivalent electrical signal (flow of current or potential difference), static or dynamic, within the operation margins of the active circuit 22000. Said means and processes for the capture and conditioning of electrical magnitudes comprise, in a generic case, filtering and scaling elements to adjust the amplitude and also the spectral content of the equivalent electrical signal. Furthermore, in the particular case in which the electrical magnitude resulting from the transduction is an electrical property of material or device, the acquisition and measurement block 22300 also comprises the means and processes, essentially based on laws constitutive of circuit elements, for the conversion of said magnitude into electrical signal. In mathematical form, if $E_X$ represents the electrical magnitude provided by the transducer x, where x is a generic index which identifies each one of the transducers comprised in section 23000; the electrical signal $V_X$ resulting from the application of the capture and conditioning techniques of the signal $E_X$, can be expressed as

$$V_X = F_X (E_X ; P_X) \qquad (6)$$

where $F_X(\cdot)$ is a bijective function which informs of the conversion and/or scaling operations leading to the obtainment of the electrical signal $V_X$, and $P_X$ denotes the possible reference parameters required by said function. In accordance with the present invention, both $F_X(\cdot)$ and $P_X$ are entities known with sufficient precision for the purposes of remote measurement with the telemetry system object of the present invention. In that sense, the entities $F_X(\cdot)$ and $P_X$ are available in the computation section 14000 of the reading instrument 10000, either in the form of mathematical operations (executable in the arithmetic logic unit 14100), or in the form of tables (stored in the internal memory 14400).

On the other hand, in accordance with the present invention, the acquisition and measurement block 22300 also provides the means and processes to extract the representative values of the measurements of the variables under observation from the equivalent electrical signal $V_X$; convert said representative values to the digital domain and transfer the resulting binary words to the processing unit 22500 for their later manipulation.

With a view to reusing devices and the unified processing of information, in accordance with the present invention, the extraction and digitization of the representative values of the measurements of the variables under observation use the clocks $CLKT_X$, generated in the timing block 22200 as reference signals.

In accordance with this, if the equivalent electrical signal $V_X$ is essentially static, its quantization shall be performed, preferably, using analogue-digital converters based on integration and digital counters. This last solution is in no way limitative of the present invention, but any other resource can be used capable of encoding the representative value of the measurement of the variable under observation using cycles of the associated time reference $CLKT_X$.

If the equivalent electrical signal $V_X$ is dynamic, the means for the extraction and digitization of the representative values of the measurements of the variables under observation shall preferably comprise means for the obtainment of a continuous value associated to the measurement; and means of quantization potentially identical to those used when the equivalent electrical signal $V_X$ is essentially static. The means for the obtainment of a dc value from the equivalent electrical signal $V_X$ ultimately depend on the type of variable monitored and are generically formed by signal-mixed circuits aided by digital processing blocks.

Thus, in accordance with the present invention, the representative value of the measurement of the variable under observation, both if the equivalent electrical signal $V_X$ is essentially static or dynamic, shall be encoded using clock cycle numbers of the associated time reference $CLKT_X$. In mathematical form, if $NT_X$ represents the number of clock cycles of the associated time reference $CLKT_X$, resulting from the processing and quantification of the equivalent electrical signal $V_X$, the following expression is fulfilled

$$NT_X = GX(V_X; FT_X; Q_X) = G_X(V_X; FTAG/MT_X; Q_X) \qquad (7)$$

where FTx is the frequency of the clock signal $CLKT_X$, $G_X(\bullet)$ is a function which informs of the quantization of the continuous level obtained from the equivalent electrical signal $V_X$ and $Q_X$ denotes the possible local parameters required by the function $G_X(\bullet)$. The function $G_X(\bullet)$ is monotone by construction and although affected by the actual resolution of the analogue-digital conversion process, it allows that the number of clock cycles $NT_X$ constitutes a univalued representative value of the measurement of the variable under observation.

Block 22400 is a storage section where a unique registration code is registered for each transponder 20000 and where it stores any parameters $Q_X$ necessary for the extraction and digitization of the representative values of the measurements of the variables under observation, in accordance with the present invention. In this block the specific adjustment parameters $C_X$, potentially derived through a calibration process of the transducers, are also stored. The storage block 22400 is preferably non-volatile, of EEPROM, EPROM, OTP type or any other permanent memory system in the absence of supply.

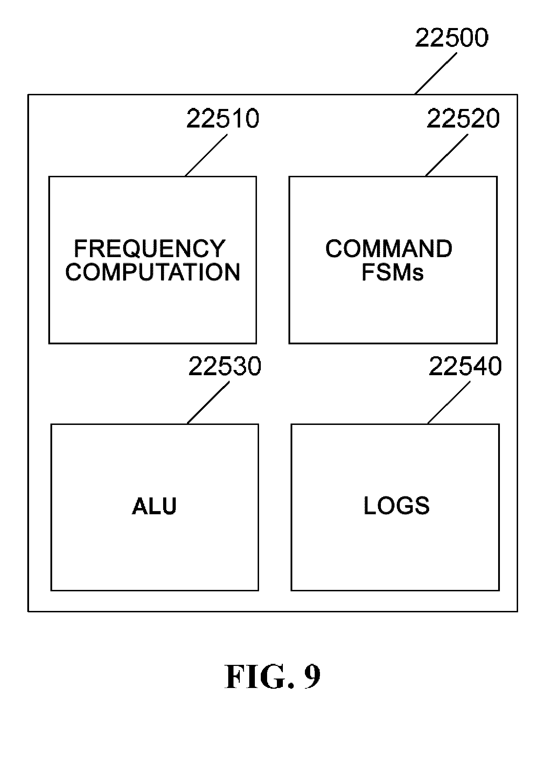

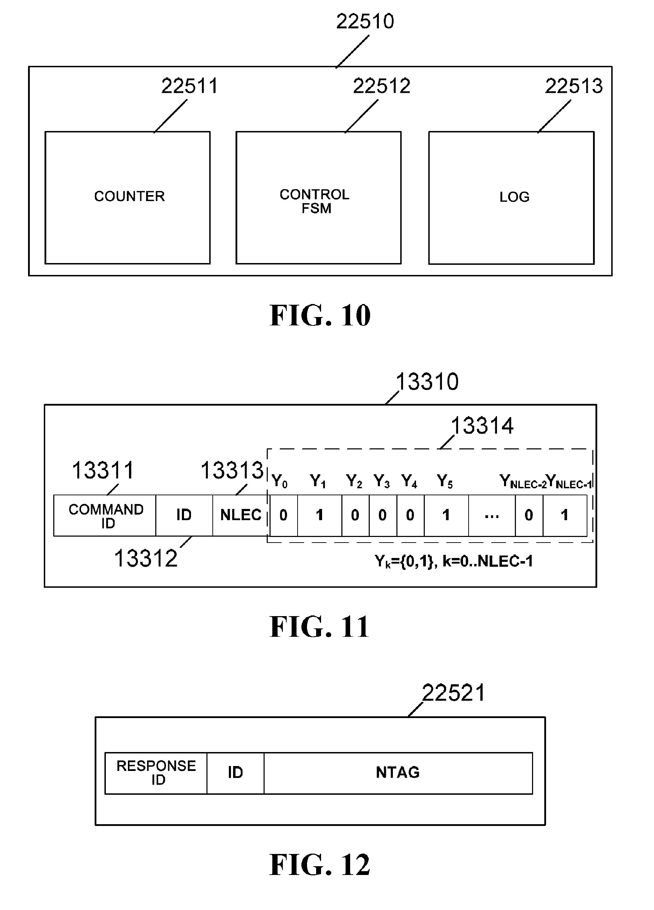

Block 22500 is a processing unit wherein the commands received from the reading instrument 10000 are interpreted; the operating modes of the different sections of the active circuit 22000 are managed to undertake the actions included in said commands; format is given to the data which must be sent to the reading instrument; and, if necessary, the data reading is performed of the storage section, of the timing section 22200, or of the sensorial signal acquisition section 22300. Figure 9 represents the block diagram of the processing unit 22500, according to an embodiment of the present invention. Said processing unit comprises a frequency computation block 22510 which is used to generate a representative value of the frequency FTAG of the reference clock CLKT for its later transmission to the reading instrument 10000; an array of finite state machines (FSMs) 22520 which are responsible for processing the commands received from the reading instrument 10000 and managing the corresponding responses; an arithmetic logic unit (ALU) 22530 to perform the necessary arithmetic operations; and an array of logs 22540 for temporary information storage and the filing of the link codes where the communication is established with the reading instrument 10000. Figure 10 shows the functional diagram of the frequency computation block 22510 according to an embodiment of the present invention. Said block comprises a counter 22511 operated by a clock CLKCO; a state machine 22512 which controls the operation of all elements of block 22510 as shall be shown below; and a log 22513 where it stores the result of the frequency computation.

Block 22600 is a signal encoding/decoding and modulation/demodulation section wherein the binary codes included in the interrogation signals are extracted from the reading instrument 10000 and wherein the data generated by the processing unit 22500 of the active circuit 22000 is modulated in accordance with the requests made by the reading instrument 10000. The encoding/decoding and modulation/demodulation block is connected to the antenna 21000 of the transponder 20000 which serves as interface with the medium.

In accordance with the signal capture and processing processes performed by the active circuit 22000 and the transducers 23000 included in the transponder 20000, in accordance with the present invention, the measurements of the variables under observation is represented in last instance using the counts of numbers of clock cycles $N_X$ of reference signals $CLKT_X$. The steps followed in these processes, expressed by the equations (3), (6) and (7), are univocal and, therefore, reversible, so that $N_X$ constitutes a representative value of the measurement of the variables under observation. Even more so, since, on the one hand, the reference parameters $P_X$ are known with sufficient precision for the purposes of remote measurement and, on the other, the adjustment parameters $C_X$ are transferred from the transceiver 20000 to the reading instrument 10000, the interpretation of the data representative of the measurements would be essentially affected by the uncertainty on the exact value of the oscillation frequency $FT_X$.

To avoid this element of uncertainty, there is the possibility of calibrating and/or adjusting circuit elements of the active block 22000, with a view to the precise synchronization of the oscillation frequency. However, this would appreciably increase the production times and cost of the wireless telemetry system. In its place, in accordance with a preferred solution of the present invention, the means and processes are provided so that the reading instrument 10000 has a precise estimate of FTAG, and thus obtain measurements of the variables monitored free from uncertainties relating to the timing of the transponder 20000.

In another aspect of the present invention, the processes are shown for the reading instrument 10000 to obtain an estimate of the frequency FTAG of the reference signal CLKT used in the active circuit 22000. The mechanism comprises four phases: the sending of a timing frame from the reading instrument 10000 to the transponder 20000; the detection and measurement of said frame in signal period units CLKCO by the active circuit 22000; the transmission of said number of periods representative of the frequency of the reference signal CLKT to the reading instrument 10000; and the calculation in this latter of the frequency FTAG based on the information received from the transponder 20000 and the known parameters of the timing frame. The processes associated to each one of these stages are described below.

In accordance with a process defined in the present invention, the first specific step for the remote estimate of the frequency FTAG consists of the synthesis of a request command for the measurement of FTAG from the signal processing section 13000 of the reading instrument 10000 to the transponder 20000. The digital sequence associated to this order 13310 is implemented in the block command generator 13300 of the signal processing section 13000 included in the reading instrument 10000. Figure 11 shows the structure of this request command 13310, in accordance with a possible embodiment of the present invention, comprising a command identifier 13311; an identifier 13312 of the wireless link established with the transponder 20000 (alternatively, it may include a code so that all transponders in the range of the reading instrument calculate an estimate of FTAG and they store it in an internal log until said information is required by the reading instrument 10000); and a value NLEC 13313 which indicates the number of bits included in a specific binary timing frame 13314, known both by the reading instrument 10000 and by the transponder 20000. The duration of the binary frame 13314 is given by

$$TLEC = NLEC0 \cdot TLEC0 + NLEC1 \cdot TLEC1 \qquad (8)$$

where TLEC0 and TLEC1 represent, respectively, the duration of the symbols '0' and '1' in accordance with the encoding used in the signal modulation and formatting section 12000 of the reading instrument 10000; and the values NLEC0 and NLEC1 represent, respectively, the number of '0' and '1' included in the frame 13314, so that NLEC = NLEC0+NLEC1. Since the time intervals TLEC0 and TLEC1 are linked to the frequency FLEC of the master clock CLKL used in the reading instrument 10000 in accordance with the equations (1) and (2), the value of TLEC can be expressed as,

$$TLEC = NLEC0*M0 + NLEC1*M1 \qquad (9)$$

and, therefore, the accuracy with which the duration of the time interval TLEC is calculated is determined by the precision of the master clock CLKL used in the reading instrument 10000, since the other parameters are whole values defined by design. Since said master clock CLKL uses, in accordance with the present invention, high precision techniques which are essentially invariable to changes in the technological process, operating temperature or supply sources, the duration TLEC has a value well defined in absolute terms. It can be highlighted that the structure of the request command 13110 is in no way limitative of the present invention but it can include any modifications necessary to fulfil the format specifications established by the communication standard between the reading instrument 10000 and the transponder 20000, provided that it satisfies the objective of sending a binary timing frame 13314.

Once the request command 13310 for the measurement of FTAG is transmitted from the reading instrument 10000 and, later, received, demodulated, decoded and identified by the transponder 20000, the following specific step for the remote estimate of the frequency FTAG, in accordance with the present invention, is the interpretation of the binary frame 13314 contained in the command 13310. The objective of this interpretation is to obtain a representative value of the duration of the frame TLEC in terms of the frequency FTAG which it is intended to estimate. This process is performed in the frequency computation section 22510 contained in the signal processing unit 22500 of the active circuit 22000. The process is as follows. When the state machine 22512 detects the start of the frame 13314 it activates the counter 22511, initially disabled. As the frame 13314 progresses, the counter 22511 dynamically updates the number of clock CLKCO cycles that have occurred and continues in this way until it receives a shutdown signal from the state machine 22512, in harmony with the finalization of the period NLEC. At that time, the counter 22511 transfers the last number of cycles measured, NTAG, to the log 22513 and passes to inactive state. Once the value of NTAG is stored, the state machine 22512 indicates to the data processing unit 22500, that the measurement has been completed and the result is ready to be sent to the reading instrument 10000. In accordance with this process the value of NTAG is given by:

$$NTAG = [TLEC*FCO] = [(TLEC*FTAG) / MCO] \qquad (10)$$

where the operator [ ] indicates rounding off of the argument towards the closest lower whole value. Since TLEC and MCO are accurately known, the number NTAG is a representative value of FTAG, and is more precise the longer the binary frame 13314, the duration whereof is TLEC, is.

The specific following step in the process for the remote estimate of the frequency FTAG is the encoding of the information NTAG in a binary sequence 22521 which, after its wireless transmission from the transponder 20000, can be recognised by the reading instrument 10000. In a possible embodiment of the present invention, said binary sequence 22521 is generated by one of the state machines 22520 contained in the processing section 22500 of the active circuit 22000, and it has the contents shown in figure 12 comprising an identifier of response type which is sent, an identifier of the wireless link established between the transponder 20000 and the reading instrument 10000 and, finally, the value of NTAG. Similarly to the request command 13110, the binary sequence 22521 may include any modifications necessary to fulfil the format specifications established by the communication standard between the reading instrument 10000 and the transponder 20000, provided that it transmits the value of NTAG.

Once the binary sequence 22521 is transmitted from the transponder 20000 and, later, received, demodulated, decoded and identified by the reading instrument 10000, the specific last step consists of the obtainment of an estimate of the transponder frequency FTAG 20000. Said estimate is performed in the computation unit 14000 of the reading instrument 10000, under the control of the signal processing section 13000, where it has the means necessary to perform the operation:

$$FTAGa = NTAG*MCO \: / \: TLEC = (NTAG*MCO) * FLEC \: / \: (NLEC0*M0 + NLEC1*M1) \qquad (11)$$

where FTAGa is the desired result of the internal frequency estimate of the transponder 20000, expressed in terms of known parameters in the reading instrument 10000 and conveniently stored in the memory 14400 of the computation unit 14000. The value FTAGa is essential to interpret the representative values, based on timing, of the variables monitored.

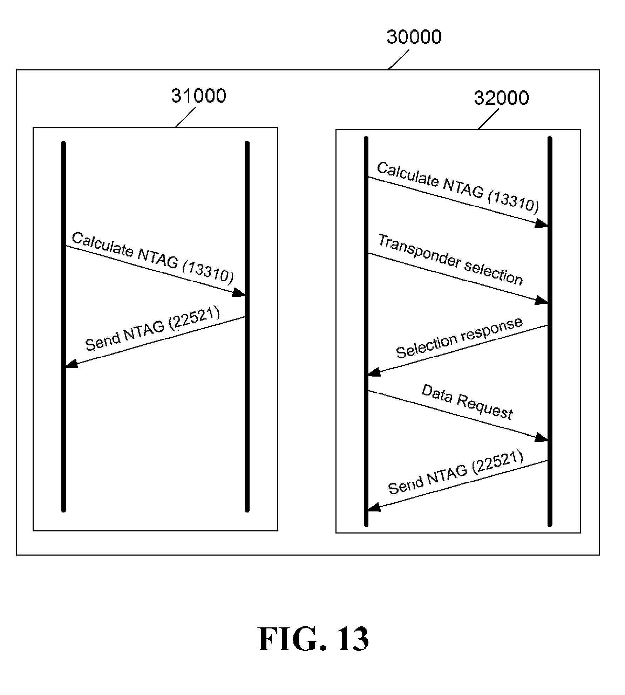

Figure 13 shows two possible scenarios of wireless communication 30000 link between the reading instrument 10000 and the transponder 20000 for the obtainment of $FTAG_a$, in accordance with the present invention.

In the first scenario 31000, the transponder 20000 immediately responds with a binary sequence 22521, after the reception of a request command 13310 from the reading instrument 10000. This scenario is only feasible when there is a single transponder 20000 enabled in the environment of the reading instrument 10000.

The second scenario 32000 starts with the emission from the reading instrument 10000 of a frequency request command FTAG 13310, on reception whereof the transponders 20000 in the range of the interrogator 10000 perform the calculation of NTAG and store it. Later, the reading instrument 10000 sends a selection command to choose a transponder from among the population of transponders in its range. The transponder 20000 selected responds with an identifier of the communication link established with the reading instrument 10000. Then, said reading instrument 10000 sends a stored data request command including, among other parameters, the communication identifier previously emitted by the selected transponder 20000. Finally, the selected transponder 20000 sends a response sequence 22521, which again includes the same communication identifier used previously. Since the link identification command is unique for each communication, the readings of the transponders 20000 in the range of the reading instrument 10000 are singularized and, therefore, collisions do not occur between the data received by the reading instrument 10000.

Following a similar process to that used for the request and later transmission of a representative value of the internal operating frequency FTAG of the active circuit 22000 described in relation to figure 13, the wireless monitoring system contemplates, in accordance with the present invention, a process for the request (direction reading instrument 10000 to transceiver 20000) and later transmission (direction transceiver 20000 to reading instrument 10000) of the value $NT_X$ representative of the measurement of the variable x monitored. The most notable difference between the two processes is that the request command of the representative value $NT_X$ emitted from the reading instrument 10000, does not contain a timing binary frame of type 13314.

In the same manner as for the remote estimate of FTAG, the particular configuration of the commands used for the reading of the measurements of the variable monitored must fulfil the format specifications established by the communication standard between the reading instrument 10000 and the transponder 20000.

In the more general case, the request and response process between an integrator 10000 and a transponder 20000, in accordance with the present invention, may contemplate the sending of more than one representative value of variable under observation. For said purpose, it must indicate the appropriate command identification 13311 in the request frame 13310 (generated by the signal processing section 13000 of the reading instrument 10000), and include the corresponding identification in the response frame 22521 (generated by the processing unit 22500 of the transponder 20000).

Since, in accordance with the present invention, the measurements of the variables under observation is represented using counts of numbers of clock cycles of reference signals CLKTx, the frequency of which can be estimated in the reading instrument 10000, in accordance with the expression

$$FT_{Xa} = (MCO / MT_X) * (NTAG / TLEC) \qquad (12)$$

and since both the functions and as parameters expressed in the equations (3), (6) and (7) are known with sufficient precision, the computation section 14000 of the reading instrument 10000 has all the elements necessary for the reproduction of the remote measurement performed in the transponder 20000.

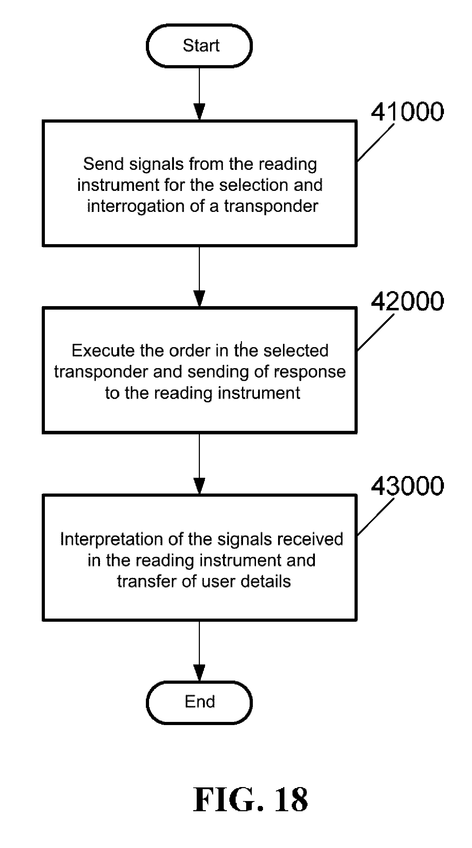

Figure 18 shows the basic steps contemplated in a communication method between a transponder "WIRELESS SENSOR" and a reading instrument "INTERROGATOR", in accordance with an example of embodiment of the present invention. The communication method between a transponder and a reading instrument, both for a wireless telemetry system for the monitoring of static and dynamic magnitudes, where the transponder is defined in any one of the examples of embodiment defined above, and where the reading instrument is also defined according to any one of the examples of embodiment mentioned above, comprises (see figure 18):

- in first place 41000, the reading instrument selects and interrogates a transponder from among a limited array of transponders located in a coverage range of the reading instrument by the sending of an interrogation signal;
- in second place 42000, the selected transponder transmits data required by the reading instrument in response to the interrogation signal received;
- in third place 43000, the reading instrument receives and interprets the data emitted by the transponder, and transfers data corresponding to the user.

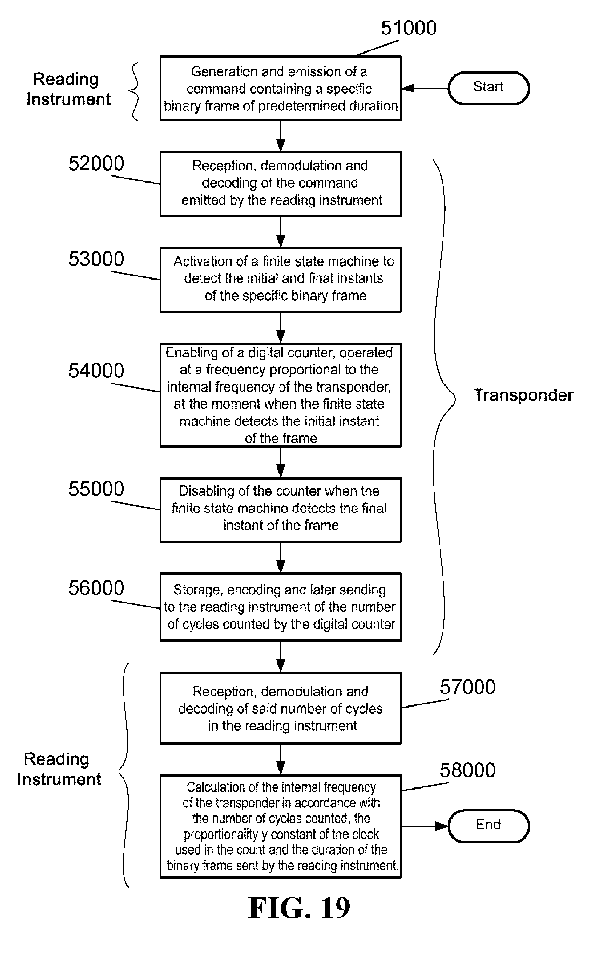

Figure 19 shows the steps contemplated in a method for the remote estimate of the internal operating frequency of a transponder "WIRELESS SENSOR" from a reading instrument "INTERROGATOR", in accordance with an example of embodiment of the present invention. The method additionally comprises remotely determining the internal operating frequency of the transponder selected and interrogated, by the reading instrument, using the following stages (see figure 19):

- generation 51000 in the reading instrument of a command containing a specific binary frame of predetermined duration;
- reception, demodulation and decoding 52000 in the transponder of the command emitted by the reading instrument;
- activation of a finite state machine 53000 to detect the initial and final instants of the specific binary frame;
- enabling of a digital counter 54000, operated at a frequency proportional to the internal frequency of the transponder, at the moment when the finite state machine detects the initial instant of the frame;
- disabling of the counter 55000 when the finite state machine detects the final instant of the frame;
- storage, encoding and later sending 56000 to the reading instrument of the number of cycles counted by the digital counter;
- reception, demodulation and decoding 57000 of said number of cycles in the reading instrument; and,
- calculation of the internal frequency 58000 of the transponder in accordance with the number of cycles counted, the proportionality constant of the clock used in the count and the duration of the binary frame sent by the reading instrument.

Implementation of the invention.

In an example of possible implementation of the present invention, the monitoring system uses RFID (Radio Frequency IDentification) technology in the UHF or microwave band, in which case the interrogator 10000 is implemented using a RFID reader conveniently configured for the application, and the transponder 20000 uses the own communication mechanisms of a RFID transponder in said operating frequencies, whereto are added the specific means and processes described in the present invention. In general, the communication system can use any other wireless "peer-to-peer" communications technology.

In this example of embodiment of the present invention, the modulation performed by block 22600 of the transponder 20000 to send data to the reading instrument 10000 uses the "backscattering" technique. In accordance with this technique, the digital transmission of information is performed using the controlled alteration of the reflection coefficients of the antenna 21000 in accordance with the binary information to send. For its implementation, the modulation section 12000 of the reading instrument 10000 must provide the means to detect the changes in the power reflected by 21000 and thus determine if the bit sent is a logical '0' or '1'.

In a preferred configuration of the example of application of the present invention, the energy management unit 22100 of the active circuit 22000 includes the means and processes to generate and store energy from the signal radiated by the reading instrument 10000 and captured by the antenna 21000 of the transponder 20000. This energy obtainment process is performed by means of a RF to DC conversion circuit, included in the unit 22100, which rectifies and multiplies the incident signal radiated until achieving a sufficiently high DC voltage to supply the transponder 20000.

In accordance with this example of embodiment, the transducers 23000 and the acquisition and measurement block 22300 of the active circuit 22000, both included in the transponder 20000, comprise the means and processes necessary for the measurement with clinical purposes of body temperature (variable under observation identified with the index 1) and heart rate (variable under observation identified with index 2) of individuals. Since, in accordance with a preferred configuration of this example of application, the transponder 20000 is passive, the power consumption generated by the means and processes comprised in blocks 23000 and 22300 for the measurement of these variables under observation is of the order of a few microamperes.

In a possible configuration of the present implementation of the invention, the electrical magnitude interpretable by the telemetry system for the measurement of body temperature (variable under observation essentially static in accordance with the time basis of the proposed monitoring system) is resistance through an exchangeable clinical thermistor (transducer included in block 23000 with zero vector C1), and the representative value of the measurement is a number which quantifies the variation of said resistance with respect to that shown by a resistor invariable with temperature which acts as parameter P1 in the equation (6). It can be highlighted that said embodiment is in no way limitative neither of the example of application nor of the present invention as a whole. Rather, any other solution which satisfies the purposes of measurement of body temperature with the precision and resolution required by the application (approximately $\pm$ 0.1°C in a temperature range around 30-45°C) may be equally valid.

Likewise, in a possible configuration of the present implementation of the invention, the electrical magnitude interpretable by the telemetry system for the measurement of heart rate of an individual (variable under dynamic observation in accordance with the time base of the proposed monitoring system) is directly the EEG signal captured using electrodes, and the representative value of the measurement is a number which quantifies the average of complex occurrences QRS in a limited time of time of a few seconds. In a preferred implementation, the detection of complexes QRS is performed in the digital domain. In any case, this preferred implementation is in no way limitative neither of the example of application nor of the present invention as a whole. Rather, any other solution which satisfies the purposes of measurement of heart rate with a precision approximately $\pm$ 1% in a range of around 30 to 240 beats per minute may be equally valid.

Also in accordance with this implementation of the present invention, the internal operating frequency FTAG of the transponder 20000 must be equal to or greater than a given value so that the tolerance levels and the time resolution of the reading instrument 10000 to transponder 20000 link, imposed by the selected RFID standard, are verified. Said frequency is internally divided, in the timing section 22200 of the active circuit 22000, by factors MCO, MT1 and MT2 with a view to generating the clocks necessary for the digital encoding of the signals representative of the internal operating frequency of the transponder 20000 (NTAG), and of the measurements of the variables under observation (NT1 and NT2).

Likewise, in accordance with this implementation of the present invention, the signal processing sections 13000 of the reading instrument 10000 and 22500 of the transponder 20000, implement the means (including state machines, logs and non-volatile memories) and obligatory processes specified in the selected RFID UHF/microwave standard. These obligatory processes are used in this example of embodiment of the present invention, not only to send the identification of the device, as is usual in RFID systems, but also to transfer information regarding the measurements of body temperature and heart rate.

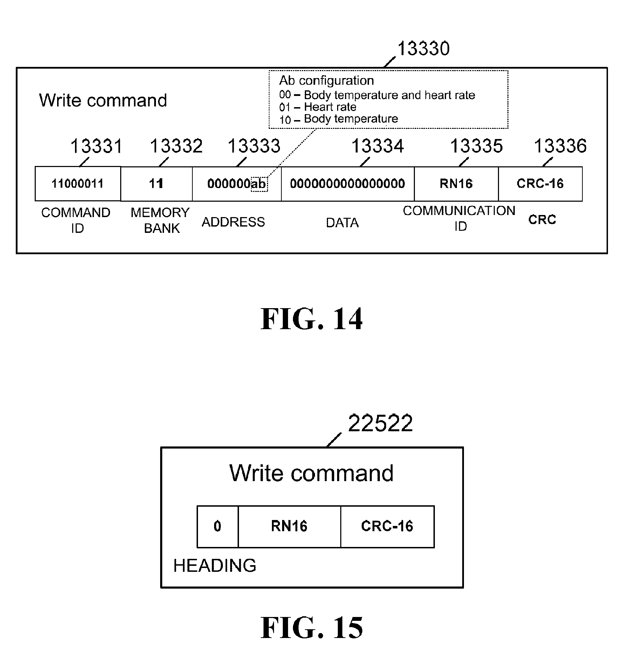

In this way, using the obligatory resources defined in the selected RFID UHF/microwave standard and available in the signal processing section 13000 of the reading instrument in accordance with the present invention, the command to indicate to the transponder 20000 the start of measurements with the sensors incorporated is performed with a WRITE-type order. Figure 14 shows the archetype of said WRITE-type command 13330, configured for the purposes of start of measurements, comprising six binary fields. The first field 13331 is an identifier established by the RFID standard. The second field 13332 makes reference to the memory bank selected. For the specific case of start of measurements in the transponder 20000, said memory bank is a user bank identified by the pair "11". The third field 13333 is a memory address pointer which in the present configuration is used to define the type of measurement to perform. In particular, depending on whether the least significant bits of the address pointer 13333 are worth "00", "01" or "10", the command orders measurement with both sensors, measurement with only the temperature sensor or measurement with only the heart rate sensor, respectively. The fourth field 13334 is a vector containing the data to write in the address noted. For the specific case of start of measurements in the transponder 20000, said vector 13334 is zero. The last two fields RN16 13335 and CRC-16 13336 are conventionally used to identify communication with the transponder 20000 and to detect errors in the link, respectively. The exact configuration of the WRITE-type command ultimately depends on the RFID protocol selected.

Also using the obligatory resources defined in the selected RFID UHF/microwave standard and available in the signal processing section 22500 of the transponder 20000 in accordance with the present invention, the transponder only responds to the WRITE-type command 13330 configured for the purposes of execution of measurements, when they have concluded. Figure 15 shows the binary frame of said response 22522 comprising a heading with value '0', as well as, the fields RN16 13335 and CRC-16 13336, previously included in the WRITE-type command. The exact configuration of the response to the WRITE-type command ultimately depends on the RFID protocol selected.

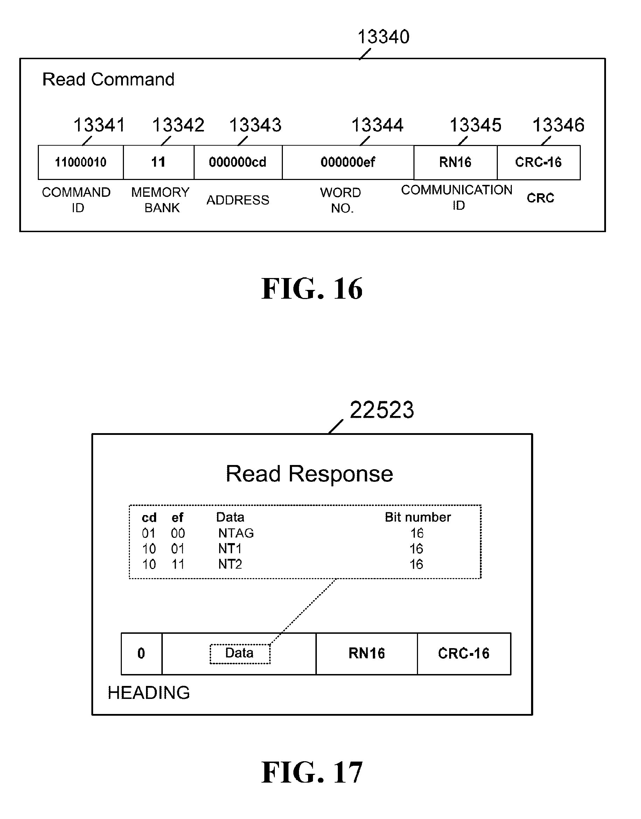

Similarly, using the obligatory resources defined in the selected RFID UHF/microwave standard and available in the signal processing section 13000 of the reading instrument in accordance with the present invention, the command to request the transponder 20000 to send the measurements taken is performed with a READ-type order. This command is used to demand the information representative both of the sensing of body temperature and heart rate, and of the operating frequency of the transponder 20000. Figure 16 shows the archetype of said READ-type command 13340, configured for the purposes of information request, comprising six binary fields. All the fields have a description similar to the corresponding fields of the WRITE-type command 13330, configured for the purposes of start of measurements. The only difference is the configuration of the least significant bits of the third 13343 and fourth fields 13344, the definitions of which determine the required data. Similarly to the WRITE-type command, the exact configuration of the READ-type command ultimately depends on the RFID protocol selected.

Following obligatory processes defined in the selected RFID UHF/microwave standard and available in the signal processing section 22500 of the transponder 20000 in accordance with the present invention, the transponder 20000 responds to the READ-type command 13340, configured for the purposes of request of information, with a binary frame compatible with the standard specifications. Figure 17 shows said frame 22523 comprising four fields. The first field is a heading whose value is '0'. The second field includes the data required by the reading instrument 10000. Said data are in harmony with the configuration of the least significant bits of the third 13343 and fourth fields 13344, defined in the READ-type command 13340. The last two fields, RN16 and CRC-16, as described above, are linked to the correct establishment of wireless communication. Again, the exact configuration of the response to the READ-type command ultimately depends on the RFID protocol selected.

In addition to the obligatory processes defined in the selected RFID UHF/microwave standard, the signal processing sections 13000 of the reading instrument 10000 and 22500 of the transponder 20000 implement, in accordance with the preferred implementation of the present invention, a specific user-defined command and compatible with the standard, for the request for information concerning the internal operating frequency of the transponder 20000. The binary frame of the specific command adopted in this example of embodiment of the present invention to make the request of a representative value of the internal operating frequency of the transponder 20000, essentially follows the same format 13310 shown in figure 11. In this example of embodiment, the value of NLEC representative of the number of bits included in the specific timing frame 13314 must take a sufficiently high value so that the error made in the estimate of the operating frequency, FTAG, of the transponder 20000, in accordance with (12), is less than 0.1 %.

In a form of embodiment of the implementation of the present invention, the antenna 21000 of the transponder 20000 is preferably planar and is preferably printed with conductive ink on a flexible substrate, adaptable to the patient's body. The connection between the antenna substrate and the active circuit 22000 is preferably performed using isotropic conductive adhesive. Since the RFID standard considered in this example of embodiment operates in the UHF/microwave band, the communications between the reading instrument 10000 and the transponder 20000 are performed in the field region far from the antenna 21000.

In a form of embodiment of the implementation of the present invention, the transponder 20000 adopts a series of protection measurements with the aim of improving the features of the sensors and reducing the impact of the environmental conditions. Thus, the assembly of the transducers 23000 and the active circuit 22000 is screened by a silicon insulating paste. Furthermore, the transponder 20000 is housed in a conventional sanitary support such as bandages, armbands, dressings or patches, or in a garment in contact with the patient's body.

The different aspects mentioned in relation to this example of embodiment of the present invention, entail novelties and improvements with respect to the state of the art, which lead to increasing the patient's comfort level and the autonomy of communications, as well as guaranteeing accuracy in the measurement and compliance with international regulations.

Thus, in accordance with this example of embodiment of the implementation of the present invention, the transponder 20000 is completely passive, i.e. external batteries are not needed, which makes it possible to considerably extend the time of useful life of the device (essentially limited by the support's resistance). This implies that not only the wireless communication section, but also the capture of physiological values, are exclusively supplied by the energy generated with the radiated signal from the reading instrument 10000. In other words, neither do the temperature and heart rate sensors require batteries, unlike the proposals of the state of the art (US2010156598 and US2004215098). Furthermore, the invention adds more features to the proposals of the state of the art (WO2011010437 and "Full Passive UHF Tag with a Temperature Sensor Suitable for Human Body Temperature Monitoring" by A. Vaz, et al), therefore, although they are devices without battery, they only allow measuring body temperature, not heart rate.

In another embodiment, the transponder 20000 shows wireless coverage ranges over three metres due to the use of far field techniques, which represents a clearly differentiating element to the other proposals. All the contributions of the state of the art cited except "Full Passive UHF Tag with a Temperature Sensor Suitable for Human Body Temperature Monitoring" by A. Vaz, et al., which specifies two metres of range, require distances between the transponder and the sensor device of a few centimetres as near field or inductive coupling techniques are used. In contrast, our invention makes it possible that the patient can be monitored at a distance and inadvertently.

Even in another aspect, this example of embodiment of the implementation of the present invention obtains usual tolerance levels in clinical applications (precision of $\pm$ 0.1°C in the window of 30-45°C, as regards the monitoring of body temperature; and of $\pm$ 1% in a range of 30 to 240 beats per minute, with respect of the heart rate monitoring) with high fidelity, since the transducers are screened and in direct contact with the individual's skin, which reduces exposure of the measurement to environmental agents or deviations induced by the operation of the active block 22000. In contrast, none of the documents of the state of the art cited explicitly contemplate the direct contact of the temperature sensor with the patient's skin. Even more so, the document of the state of the art US2010171596 does not include any sensor nor is it compatible with commercial RFID readers.

Finally, in this example of embodiment of the implementation of the present invention no calibration or adjusting techniques are used, unlike the disclosures of the state of the art in WO2011010437, "Full Passive UHF Tag with a Temperature Sensor Suitable for Human Body Temperature Monitoring" by A. Vaz, et al. and US2004215098 which require calibration to guarantee that the parameter monitored has the desired precision. Generally, the calibration process must be individually performed on each transponder once the assembly process has concluded. In a mass manufacturing scenario, the calibration process slows down the production volume, it may require expensive machinery and add a significant cost to the end value of the transponder. In the present invention, neither the sensors nor the RFID communications unit require calibration or adjusting of discrete devices, which appreciably reduces production times and cost. The contribution of the document of the state of the art US2010156598 has a high degree of lack of definition since it neither describes the sensors used nor considers a signal acquisition diagram.

In accordance with the above, the individual may be remotely monitored, inadvertently for this, which opens the doors to new applications such as the instrumentalization of hospital beds (from a single control post it could be possible to monitor the temperature and heart rate of an entire floor), the control of patients in triage or the monitoring of the vital signs of the elderly in old people's homes.

Likewise, this example of implementation of the present invention makes it possible to complement/improve the functionality of devices on the market such as baby monitors (at present they provide audio and, in some cases, video and movement control). Having information on the baby's temperature and heart rate would provide added value to these services.

In another application, this example of embodiment of the implementation of the present invention can be used in a system for the monitoring of the stress of an individual, since both the body temperature and the heart rate are essential indications of stress.

**Claims**

1. Reading instrument for a wireless telemetry system for the monitoring of static and dynamic magnitudes, where the reading instrument (10000) is **characterized in that** it comprises:

    • an antenna (11000) capable of transmitting different interrogation signals towards a transponder and of receiving information signals from said transponder;
    • a communication section (12000) connected to the antenna, which provides the means and processes to define signal formats, a data encoding, a modulation type, transmission/reception rates, a RF envelope and parameters which establish the wireless communication with the transponder;
    • a signal processing section (13000), which is connected to the communication section (12000), where the signal processing section (13000) provides the means and processes necessary to create, in accordance with the user's instructions, the command sequence required to select and interrogate the transponder, as well as to identify and classify the data received from said transponder;
    • a computation section (14000) which interprets the values representative of the internal operating frequency of the transponder and of the measurements of the variables under observation, previously classified in the signal processing section (13000);
    • a timing unit (15000) stable with respect to temperature, which determines the operating frequency of the entire reading instrument and which provides the computation section (14000) with the parameters necessary to interpret the representative values both of the internal operating frequency of the transponder and the measurements of the variables under observation;

• a user interface section (16000) which provides the means to activate the identification and measurement processes of variables under observation from the transponder; and,

• a data output section (17000) which provides means and processes to store the data measured by the transponder and communication means of said data with the exterior.

2. Transponder for a wireless telemetry system for the monitoring of static and dynamic magnitudes, where the transponder (20000) is **characterized in that** it comprises:

• an antenna (21000) capable of receiving different interrogation signals from a reading instrument and of transmitting to said reading instrument signals representative of their exclusive identification and of their internal operating frequency, as well as of measurements of variables under observation;

• an active circuit (22000) which manages communication operations with the reading instrument as well as data processing operations in accordance with the instructions received; and,

• an array of transducers (23000), associated to the variables under observation, which convert monitored magnitudes of the variables under observation to electrical values which can be interpreted by the active circuit, so that the conversion performed by each transducer is a monotone function, known and available in the computation section of the reading instrument.

3. Transponder for a wireless telemetry system for the monitoring of static and dynamic magnitudes, according to claim 2, wherein the active circuit (22000) comprises:

• a storage section (22100) wherein a unique identification code is registered for the transponder;

• an energy generation and storage unit (22400) wherein supply voltages necessary for the transponder operation are generated and regulated;

• a timing section (22200) wherein clock signals necessary to activate the transponder are autonomously generated;

• a signal modulation and demodulation section (22600) wherein binary codes included in the interrogation signals are extracted from the reading instrument, and wherein the data generated by the transponder are modulated in accordance with the requests made by said reading instrument;

• a processing unit (22500) wherein the commands received from the reading instrument are interpreted, the operating modes of the different sections of the active circuit are managed to carry out actions included in said commands and format is given to the binary numbers representative of the internal operating frequency of the transponder and of the measurements of the variables under observation which must be sent to said reading instrument; and,

• an acquisition and measurement section (22300) which provides the means and processes to sequence the activity of the transducers, convert the analogue signals obtained from said transducers to the digital domain and transfer said digital data to the processing unit, so that the data conversion performed in the acquisition and measurement section is a monotone function, which is known and available in the computation section of the reading instrument.

4. Transponder for a wireless telemetry system for the monitoring of static and dynamic magnitudes, according to claim 3, wherein the active circuit (22000) is manufactured on a substrate selected from among: silicon, organic, silicon on insulator, silicon-germanium, indium phosphide and gallium arsenide.

5. Transponder for a wireless telemetry system for the monitoring of static and dynamic magnitudes, according to claim 3, wherein the processing unit (22500) comprises a finite state machine installed in the transponder which detects a timing frame sent from the reading instrument, and a digital counter activated by an internal clock of the transponder which counts the number of clock periods comprised in said frame.

6. Communication method between a transponder and a reading instrument, both for a wireless telemetry system for the monitoring of static and dynamic magnitudes, where the transponder is defined in any of claims 2 to 5, and where the reading instrument is defined according to claim 1; the communication method is **characterized in that** it comprises:

• in first place (41000), the reading instrument selects and interrogates a transponder from among a limited array of transponders located in a coverage range of the reading instrument by the sending of an interrogation signal;

• in second place (42000), the selected transponder transmits data required by the reading instrument in response to the interrogation signal received;
• in third place (43000), the reading instrument receives and interprets the data emitted by the transponder, and transfers data corresponding to the user.

7. Communication method between a transponder and a reading instrument, according to claim 6, where the method additionally comprises remotely determining the internal operating frequency of the transponder selected and interrogated, by the reading instrument, using the following stages:

• generation (51000) in the reading instrument of a command containing a specific binary frame of predetermined duration;
• reception, demodulation and decoding (52000) in the transponder of the command emitted by the reading instrument;
• activation of a finite state machine (53000) to detect the initial and final instants of the specific binary frame;
• enabling of a digital counter (54000), operated at a frequency proportional to the internal frequency of the transponder, at the moment when the finite state machine detects the initial instant of the frame;
• disabling of the counter (55000) when the finite state machine detects the final instant of the frame;
• storage, encoding and later sending (56000) to the reading instrument of the number of cycles counted by the digital counter;
• reception, demodulation and decoding (57000) of said number of cycles in the reading instrument; and,
• calculation of the internal frequency (58000) of the transponder in accordance with the number of cycles counted, the proportionality constant of the clock used in the count and the duration of the binary frame sent by the reading instrument.

8. Wireless telemetry system for the monitoring of static and dynamic magnitudes comprising a transponder in accordance with any of claims 2 to 5, a reading instrument according to claim 1 and a method for the remote determination of the internal operating frequency of the transponder using the reading instrument according to any of claims 6 to 7.

10000

20000

INTERROGATOR

WIRELESS SENSOR

**FIG. 1**

**FIG. 2**

13000

13100

CONFIGURATION
LOG

13300

COMMAND
GENERATOR

13200

PROCESSOR

13400

DATA
I/O

# FIG. 3

14000

14100

14300

ALU

RESULT
LOG

14200

14400

OPERATION
LOGS

MEMORY

**FIG. 4**

CLKL

Symbol '0'

Symbol '1'

(M0=8)

(M1=16)

**FIG. 5**

EP 2 879 075 A1

20000

21000                          22000

ANTENNA        ACTIVE CIRCUIT
                  FOR
               PROCESSING
                  AND
              COMMUNICATION

23000

TRANSDUCERS FOR THE
VARIABLES MONITORED

FIG. 6

24

22000

| ENERGY MANAGEMENT<br>22100 | TIMING<br>22200 | SIGNAL ACQUISITION<br>22300 |
|---|---|---|
| STORAGE MEMORY<br>22400 | PROCESSING<br>22500 | SIGNAL DE/ MODULATION DE/ CODING<br>22600 |

**FIG. 7**

CLKT

CLKT1

CLKT2

**FIG. 8**

22500

22510

22520

FREQUENCY
COMPUTATION

COMMAND
FSMs

22530

22540

ALU

LOGS

**FIG. 9**

22510

| COUNTER | CONTROL FSM | LOG |
|---------|-------------|-----|

22511    22512    22513

## FIG. 10

13310

13314

13311        13313

| COMMAND ID | ID | NLEC | $Y_0$ | $Y_1$ | $Y_2$ | $Y_3$ | $Y_4$ | $Y_5$ | ... | $Y_{NLEC-2}$ | $Y_{NLEC-1}$ |
|------------|----|----|-----|-----|-----|-----|-----|-----|-----|-----|-----|
|            |    |      | 0 | 1 | 0 | 0 | 0 | 1 | ... | 0 | 1 |

13312

$Y_k = \{0,1\}, k = 0..NLEC-1$

## FIG. 11

22521

| RESPONSE ID | ID | NTAG |
|-------------|----|----|

## FIG. 12

**FIG. 13**

13330

Write command

Ab configuration
00 – Body temperature and heart rate
01 – Heart rate
10 – Body temperature

| 13331 | 13332 | 13333 | | 13334 | 13335 | 13336 |
|---|---|---|---|---|---|---|
| 11000011 | 11 | 000000ab | 0000000000000000 | RN16 | CRC-16 | |
| COMMAND ID | MEMORY BANK | ADDRESS | DATA | COMMUNICATION ID | CRC | |

## FIG. 14

22522

Write command

| 0 | RN16 | CRC-16 |
|---|---|---|

HEADING

## FIG. 15

13340

| 11000010 | 11 | 000000cd | 000000ef | RN16 | CRC-16 |
|---|---|---|---|---|---|
| COMMAND ID | MEMORY BANK | ADDRESS | WORD NO. | COMMUNICATION ID | CRC |

Read Command

13341   13342   13343      13344      13345   13346

**FIG. 16**

22523

Read Response

| cd | ef | Data | Bit number |
|---|---|---|---|
| 01 | 00 | NTAG | 16 |
| 10 | 01 | NT1 | 16 |
| 10 | 11 | NT2 | 16 |

| 0 | Data | RN16 | CRC-16 |
|---|---|---|---|

HEADING

**FIG. 17**

Start

Send signals from the reading
instrument for the selection and
interrogation of a transponder

41000

Execute the order in the selected
transponder and sending of response
to the reading instrument

42000

Interpretation of the signals received
in the reading instrument and
transfer of user details

43000

End

FIG. 18

51000

Reading
Instrument

Generation and emission of a
command containing a specific
binary frame of predetermined duration

Start

52000

Reception, demodulation and
decoding of the command
emitted by the reading instrument

53000

Activation of a finite state machine to
detect the initial and final instants
of the specific binary frame

54000

Enabling of a digital counter, operated
at a frequency proportional to the
internal frequency of the transponder,
at the moment when the finite state
machine detects the initial instant
of the frame

55000

Disabling of the counter when the
finite state machine detects the final
instant of the frame

Transponder

56000

Storage, encoding and later sending
to the reading instrument of the number
of cycles counted by the digital counter

57000

Reception, demodulation and
decoding of said number of cycles
in the reading instrument

Reading
Instrument

58000

Calculation of the internal frequency
of the transponder in accordance with
the number of cycles counted, the
proportionality y constant of the clock
used in the count and the duration of the
binary frame sent by the reading instrument.

End

## FIG. 19

<table>
<tr><td colspan="2" align="center">**INTERNATIONAL SEARCH REPORT**</td><td>International application No.<br><br>PCT/ES2013/070501</td></tr>
</table>

**A. CLASSIFICATION OF SUBJECT MATTER**

*G06K7/00* (2006.01)

According to International Patent Classification (IPC) or to both national classification and IPC

**B. FIELDS SEARCHED**

Minimum documentation searched (classification system followed by classification symbols)
G06K

Documentation searched other than minimum documentation to the extent that such documents are included in the fields searched

Electronic data base consulted during the international search (name of data base and, where practicable, search terms used)

EPODOC, INVENES, WPI, NPL, XPESP, XPAIP, XPI3E, INSPEC.

**C. DOCUMENTS CONSIDERED TO BE RELEVANT**

| Category* | Citation of document, with indication, where appropriate, of the relevant passages | Relevant to claim No. |
|---|---|---|
| A | US 2011221569 A1 (HAMEL MICHAEL JOHN ET AL.) 15/09/2011, paragraphs [0008] a [0114]; figures 1 a 19. | 1-8 |
| A | Dalola S. et al.. "Autonomous Sensor System With Power Harvesting for Telemetric Temperature Measurements of Pipes". IEEE Transactions on Instrumentation and Measurement, 20090501 IEEE Service Center, Piscataway, NJ, US 01/05/2009 Vol: 58 No: 5 Pags: 1471 - 1478 XP011251004 ISSN 0018-9456 . | 1-8 |

☐ Further documents are listed in the continuation of Box C.        ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search 09/12/2013 | Date of mailing of the international search report **(17/12/2013)** |
|---|---|
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>J. Botella Maldonado<br><br><br>Telephone No. 91 3495382 |

Form PCT/ISA/210 (second sheet) (July 2009)

INTERNATIONAL SEARCH REPORT

Information on patent family members

International application No.

PCT/ES2013/070501

| Patent document cited in the search report | Publication date | Patent family member(s) | Publication date |
|---|---|---|---|
| US2011221569 A1 | 15.09.2011 | NONE | |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011010437 A1
- US 2010156598 A1
- US 2010171596 A1
- US 2004215098 A1

- US 2010156598 A
- US 2004215098 A
- WO 2011010437 A
- US 2010171596 A

### Non-patent literature cited in the description

- **A. VAZ et al.** Full Passive UHF Tag with a Temperature Sensor Suitable for Human Body Temperature Monitoring. *IEEE Trans. on Circuits and Systems II - Express Briefs,* vol. 57 (2), 95-99